(19) Europäisches Patentamt
European Patent Office
Office européen des brevets

(11) Numéro de publication: **0 086 723**
**B1**

# (12) FASCICULE DE BREVET EUROPEEN

(45) Date de publication du fascicule du brevet: **22.04.87**

(21) Numéro de dépôt: **83400314.7**

(22) Date de dépôt: **15.02.83**

(51) Int. Cl.⁴: **C 07 D 487/04,** C 07 D 405/12, A 61 K 31/505 // (C07D487/04, 241:00, 239:00)

(54) **Nouvelles pyrimidones substituées et leurs sels, leur préparation, leur application comme médicaments, les compositions les renfermant et les nouveaux intermédiaires obtenus.**

(30) Priorité: **17.02.82 GB 8204634**

(43) Date de publication de la demande: **24.08.83 Bulletin 83/34**

(45) Mention de la délivrance du brevet: **22.04.87 Bulletin 87/17**

(84) Etats contractants désignés: **BE CH DE FR IT LI NL**

(56) Documents cités:
**DE - A - 2 513 930**
**FR - A - 2 432 520**

(73) Titulaire: **ROUSSEL-UCLAF, 35, boulevard des Invalides, F-75007 Paris (FR)**

(72) Inventeur: **Westwodd, Robert, 8, Fernham Road, Faringdon Oxfordshire (GB)**
Inventeur: **Miller, Peter, 88, Kingshill Road, Swindon Wiltshire (GB)**
Inventeur: **Ager, Ian Robert, Three Willows Gosditch Ashton Keynes, Swindon Wilt shire (GB)**
Inventeur: **Kay, David Paul, 4, Church Path, Purton Wiltshire (GB)**

(74) Mandataire: **Vieillefosse, Jean-Claude et al, Département des Brevets ROUSSEL UCLAF B.P no 9, F-93230 Romainville (FR)**

EP 0 086 723 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouvelles pyrimidones substituées et leurs sels, ainsi que le procédé de préparation, l'application à titre de médicaments de ces nouveaux produits, les compositions les renfermant et les nouveaux intermédiaires obtenus.

L'invention a pour objet de nouvelles pyrimidones substituées ainsi que leurs sels, caractérisées en ce qu'elles répondent à la formule générale (I):

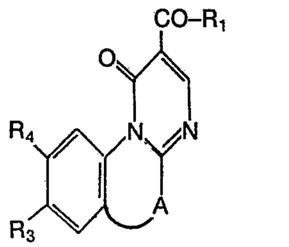

(I)

dans laquelle $R_1$ représente un radical hydroxy, un radical alcoxy renfermant de 1 à 5 atomes de carbone, un radical (1H-tétrazol-5-yl)amino, un radical hydrazino,

A représente un groupement 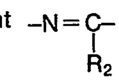

dans lequel $R_2$ représente un atome d'hydrogène ou un radical alcoyle renfermant de 1 à 5 atomes de carbone, étant entendu que $R_2$ ne peut représenter un atome d'hydrogène lorsque $R_1$ représente un radical alcoxy renfermant de 1 à 5 atomes de carbone, ou

A représente un groupement 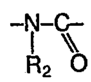

dans lequel $R_2$ a la signification déjà indiquée,

$R_3$ et $R_4$, identiques ou différents, représentent un atome d'hydrogène, un atome d'halogène, un radical alcoyle renfermant de 1 à 5 atomes de carbone ou un radical alcoxy renfermant de 1 à 5 atomes de carbone.

Dans la formule générale I et dans ce qui suit, le terme radical alcoxyl renfermant de 1 à 5 atomes de carbone désigne par exemple, un radical méthoxy, éthoxy, propoxy, isopropoxy ou butoxy, le terme radical alcoyle renfermant de 1 à 5 atomes de carbone désigne, par exemple, un radical méthyle, éthyle, propyle, isopropyle, butyle ou pentyle, le terme atome d'halogène désigne, par exemple, un atome de chlore, de fluor ou de brome.

Les produits de formule I dans laquelle $R_1$ ne représente pas un radical hydroxy présentent un caractère basique et peuvent par conséquent former des sels d'addition avec les acides tels que, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcanesulfoniques, tels que l'acide méthane sulfonique et aryl sulfoniques, tels que l'acide benzène sulfonique.

Les produits de formule I dans laquelle $R_1$ représente un radical hydroxy présentent un caractère acide, dans ce cas, les sels peuvent être des sels métalliques ou des sels d'addition avec les bases azotées.

Les sels métalliques peuvent être par exemple, les sels de métaux alcalins, tels le sodium, le potassium ou le lithium, de métaux alcalino-terreux, tels que le calcium ou de métaux tels l'aluminium ou le magnésium.

Les sels de bases azotées peuvent être par exemple, les sels d'ammonium ou les sels d'amines, telles la trométhamine, la lysine, l'arginine, la triéthanolamine.

Parmi les produits, objet de l'invention, on peut citer notamment les dérivés répondant à la formule (I) ci-dessus, ainsi que leurs sels, caractérisés en ce que dans ladite formule (I), $R_1$ et A ont la signification déjà indiquée et $R_3$ et $R_4$, identiques ou différents, représentent un atome d'hydrogène, un atome de chlore, un radical méthoxy ou méthyle.

Parmi ces derniers, on peut citer les dérivés répondant à la formule (I) ci-dessus, ainsi que leurs sels, caractérisés en ce que $R_1$ a la signification déjà indiquée,

A représente un groupement 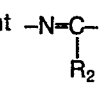

dans lequel $R_2$ représente un atome d'hydrogène ou un radical méthyle, étant entendu que $R_2$ ne peut représenter un atome d'hydrogène lorsque $R_1$ représente un radical alcoxy,

$R_2$ et $R_4$, identiques ou différents, représentent un atome d'hydrogène ou de chlore, un radical méthoxy ou méthyle, et, notamment l'acide 1-oxo 1H-pyrimido[1,2-a]quinoxalin-2-carboxylique et ses sels, le N-(5-tétrazolyl)1-oxo-1H-pyrimido[1,2-a]quinoxalin-2-carboxamide et ses sels.

Parmi les produits, objet de l'invention, on peut retenir également les dérivés répondant à la formule (I) ci-dessus, ainsi que leurs sels, caractérisés en ce que $R_1$ a la signification déjà indiquée,

A représente un groupement 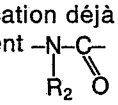

dans lequel $R_2$ représente un atome d'hydrogène ou un radical méthyle,

$R_3$ et $R_4$ identiques ou différents, représentent un atome d'hydrogène ou de chlore, un radical méthoxy ou méthyle et notamment l'acide 1,5-dioxo-1,6-dihydropyrimido[1,2-a]quinoxalin-2-carboxylique et ses sels.

L'invention a également pour objet un procédé de préparation des produits répondant à la formule générale (I) tels que définis ci-dessus, ainsi que de leurs sels, caractérisé en ce que l'on fait réagir un produit de formule (II):

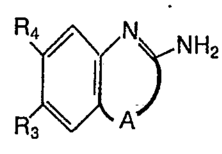

(II)

dans laquelle A, $R_3$ et $R_4$ ont la signification déjà indiquée, avec un produit de formule (III):

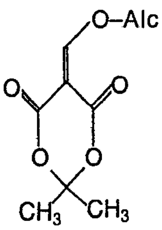

(III)

dans laquelle Alc représente un radical alcoyle renfermant de 1 à 5 atomes de carbone, pour obtenir un produit de formule (IV):

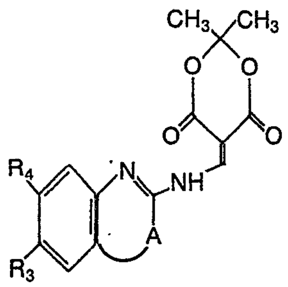

(IV)

dans laquelle A, $R_3$ et $R_4$ ont la signification déjà indiquée, puis cyclise ledit produit de formule (IV), pour obtenir un produit de formule ($I_A$):

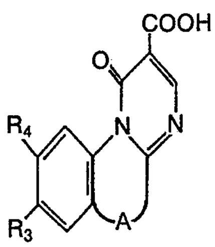

($I_A$)

dans laquelle A, $R_3$ et $R_4$ ont la signification déjà indiquée, et que:

– soit on isole et, si désiré, salifie ledit produit de formule ($I_A$) ainsi obtenu,

– soit on fait réagir ledit produit de formule ($I_A$) ou un dérivé fonctionnel de ce dernier, avec un produit de formule (V):

$$H\text{–}R'_1 \qquad (V)$$

dans laquelle $R'_1$ a la même signification que R, à l'exclusion du radical hydroxy, pour obtenir un produit de formule ($I_B$):

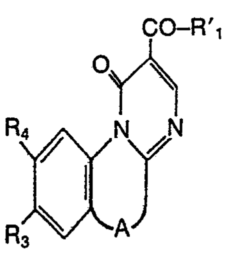

($I_B$)

dans laquelle A, $R'_1$, $R_3$ et $R_4$ ont la signification déjà indiquée, que l'on peut salifier, si désiré.

Dans des conditions préférentielles de mise en œuvre de l'invention, le procédé de préparation ci-dessus décrit est caractérisé en ce que:

a) la réaction du produit de formule (II) avec le produit de formule (III) est effectuée au sein d'un solvant organique tel que le méthanol;

b) la cyclisation du produit de formule (IV) est effectuée par chauffage du milieu réactionnel au reflux du mélange de 1 à 5 heures;

c) le dérivé fonctionnel du produit de formule ($I_A$) est l'anhydride, un anhydride mixte, un ester, ou avantageusement le chlorure d'acide. De tels dérivés fonctionnels peuvent être préparés par des méthodes conventionnelles, éventuellement en opérant in situ. Lorsque l'acide de formule $I_A$ est utilisé, il est en général avantageux d'opérer en présence d'un agent activateur et/ou d'un agent déshydratant, tel que le carbonyldiimidazole. La réaction est avantageusement effectuée en présence d'un solvant tel que le diméthylformamide.

L'invention a également pour objet une variante du procédé de préparation des produits répondant à la formule générale (I), ci-dessus, dans laquelle $R_3$ et $R_4$ ont la signification déjà indiquée, $R_1$ représente un radical alcoxy renfermant de 1 à 5 atomes de carbone, caractérisée en ce que l'on fait réagir un produit de formule (II):

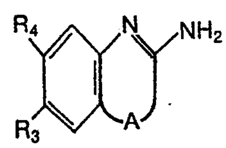

(II)

dans laquelle $R_3$ et $R_4$ ont la signification déjà indiquée, avec un éthoxy méthylène malonate de dialcoyle, pour obtenir un produit de formule (VI):

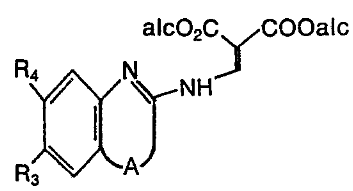

(VI)

dans laquelle A, $R_3$ et $R_4$ ont la signification déjà indiquée, et alc représente un radical alcoyle renfermant de 1 à 5 atomes de carbone, puis cyclise par chauffage le produit de formule (VI) obtenu, pour obtenir le produit de formule I' recherché:

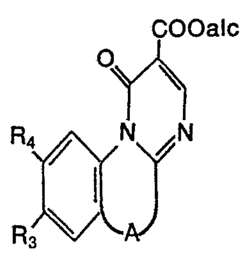

(I')

dans laquelle A, $R_3$, $R_4$ et alc ont la signification déjà indiquée, correspondant à un produit de formule I dans laquelle $R_1$ représente un radical alcoxy, que l'on peut salifier, si désiré.

Dans des conditions préférentielles de mise en œuvre de l'invention, cette variante est caractérisée en ce que:

a) la réaction du produit de formule (II) avec le malonate est effectuée au sein d'un solvant organique tel que le xylène;

b) la cyclisation du produit de formule IV est effectuée par chauffage ou en utilisant un agent de condensation tel que l'acide polyphosphorique.

Les produits de formule I peuvent être salifiés selon les méthodes usuelles. Ainsi, lorsque $R_1$ représente un radical hydroxy, on peut faire réagir les produits de formule I correspondants avec une base; lorsque $R_1$ est différent d'un radical hydroxy, on peut faire réagir les produits de formule I correspondants avec un acide.

Les produits, objet de la présente invention, ainsi que leurs sels, possèdent de très intéressantes propriétés pharmacologiques; ils sont doués notamment de remarquables propriétés antiallergiques.

Ces propriétés sont illustrées plus loin dans la partie expérimentale.

Ces propriétés justifient l'utilisation des nouvelles pyrimidones substituées, objet de la présente invention, ainsi que de leurs sels, à titre de médicaments.

La présente demande a ainsi également pour objet l'application à titre de médicaments des nouvelles pyrimidones substituées telles que définies par la formule (I) ainsi que de leurs sels pharmaceutiquement acceptables.

Parmi les médicaments, objet de l'invention, on retient de préférence, les médicaments caractérisés en ce qu'ils sont constitués par les dérivés répondant à la formule (I) dans laquelle $R_1$ et A ont la signification déjà indiquée et

$R_3$ et $R_4$, identiques ou différents, représentent un atome d'hydrogène, un atome de chlore, un radical méthoxy ou méthyle ainsi que leurs sels pharmaceutiquement acceptables.

Parmi ceux-ci, on retient notamment les médicaments, caractérisés en ce qu'ils sont constitués par les dérivés répondant à la formule (I) dans laquelle $R_1$ a la signification déjà indiquée,

A représente un groupement 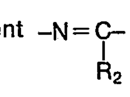

dans lequel $R_2$ représente un atome d'hydrogène ou un radical méthyle, étant entendu que $R_2$ ne peut représenter un atome d'hydrogène lorsque $R_1$ représente un radical alcoxy,

$R_3$ et $R_4$, identiques ou différents, représentent un atome d'hydrogène ou de chlore, un radical méthoxy ou méthyle ainsi que leurs sels pharmaceutiquement acceptables.

Parmi ces derniers, on retient tout particulièrement les produits dont les noms suivent:

– l'acide 1-oxo 1H-pyrimido[1,2-a]quinoxalin-2-carboxylique et ses sels pharmaceutiquement acceptables,

– le N-(5-tétrazolyl)1-oxo-1H-pyrimido[1,2-a]quinoxalin-2-carboxamide et ses sels pharmaceutiquement acceptables.

Parmi les médicaments, objet de l'invention, on retient également ceux caractérisés en ce qu'il sont constitués par les dérivés répondant à la formule (I) dans laquelle $R_1$ a la signification déjà indiquée;

A représente un groupement 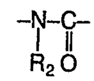

dans lequel $R_2$ représente un atome d'hydrogène ou un radical méthyle,

$R_3$ et $R_4$, identiques ou différents, représentent un atome d'hydrogène ou de chlore, un radical méthoxy ou méthyle, ainsi que leurs sels pharmaceutiquement acceptables, et notamment le produit dont le nom suit:

– l'acide 1,5-dioxo-1,6-dihydropyrimido[1,2-a]-quinoxalin-2-carboxylique et ses sels pharmaceutiquement acceptables.

Ces médicaments trouvent, par exemple, leur emploi dans le traitement de l'asthme allergique et des bronchites asthmatiformes d'origine allergique.

La dose usuelle variable selon le produit utilisé, le sujet traité et l'affection en cause peut être, par exemple, de 0,25 mg à 100 mg par jour, par voie orale chez l'homme.

L'invention a enfin pour objet les compositions pharmaceutiques qui renferment au moins un dérivé précité ou l'un de ses sels pharmaceutiquement acceptables, à titre de principe actif.

A titre de médicaments, les dérivés répondant à la formule I et leurs sels pharmaceutiquement acceptables peuvent être incorporés dans des compositions pharmaceutiques destinées à la voie digestive, parentérale ou locale.

Ces compositions pharmaceutiques peuvent être par exemple solides ou liquides et se présenter sous les formes pharmaceutiques couramment utilisées en médecine humaine, comme, par exemple, les comprimés, simples ou dragéifiés, les gélules, les granulés, les suppositoires, les sirops, les aérosols, les crèmes, les pommades, les préparations injectables, elles sont préparées selon les méthodes usuelles. Le ou les principes actifs peuvent y être incorporés à des excipients habituellement employés dans ces compositions pharmaceutiques, tels que le talc, la gomme arabique, la lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet à titre de produits industriels nouveaux, utiles notamment pour la préparation des produits de formule I, les produits de formule (IV):

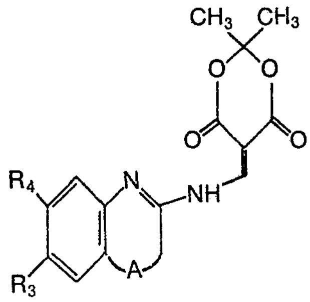

(IV)

dans laquelle A, $R_3$ et $R_4$ ont la signification déjà indiquée.

Il va être donné maintenant à titre non limitatif, des exemples de mise en œuvre de l'invention.

Les exemples suivants illustrent l'invention sans toutefois la limiter.

Exemple 1: Acide 1-oxo 1H-pyrimido[1,2-a]-quinoxalin-2-carboxylique.

Stade A: N-(2'-quinoxalinyl)amino méthylène malonate d'isopropylidène.

On ajoute une solution de 2 g de méthoxyméthylène malonate d'isopropylidène dans 40 cm³ de méthanol à une solution de 2 g de 2-aminoquinoxaline dans 40 cm³ de méthanol. On agite, filtre le précipité formé et le lave au méthanol. On obtient 2,8 g de produit attendu.

Stade B: Acide 1-oxo 1H-pyrimido[1,2-a]-quinoxalin-2-carboxylique.

On dissout 1,6 g de produit obtenu au Stade A dans 50 cm³ de chlorure et méthylène et ajoute 50 cm³ d'acide polyphosphorique. On chauffe le mélange à 60 °C pour éliminer le solvant, puis poursuit le chauffage pendant une heure. On refroidit, dilue à l'eau, filtre le précipité formé, le lave à l'eau et le recristallise dans un mélange chloroforme-méthanol. On obtient 1,21 g de produit attendu.

F = 216–218 °C.

Exemple 2: N-(5-tétrazolyl)1-oxo 1H-pyrimido[1,2-a]quinoxalin-2-carboxamide.

On chauffe au bain-marie pendant 15 minutes, un mélange de 250 mg de produit obtenu à l'exemple 1 et 200 mg de carbonyl diimidazole dans 5 cm³ de diméthylformamide, puis ajoute 100 mg de 5-amino tétrazole. On chauffe à nouveau pendant 30 minutes, puis verse dans l'eau et maintient à température ambiante pendant une heure. On filtre les cristaux formés, les lave à l'eau puis à l'éther et les sèche. On obtient 240 mg de produit attendu. F > 300 °C.

Exemples 3 à 10.

Stade A: En utilisant une méthode analogue à celle décrite à l'exemple 1, mais en partant du composé de départ approprié, dans lequel A, $R_2$, $R_3$ et $R_4$ ont les significations indiquées dans le tableau ci-après, on a préparé les produits des exemples 3, 4, 6, 7, 8 et 10. (Voir Tableau ci-après).

Stade B: En utilisant une méthode analogue à celle décrite à l'exemple 2, mais en partant de l'acide de départ approprié, dans lequel A, $R_3$ et $R_4$ ont les significations indiquées dans le tableau ci-après, on a préparé les produits des exemples 5 et 9. (Voir Tableau ci-après).

| Exemple • | $R_1$ | A | $R_2$ | $R_3$ | $R_4$ | M. pt. | Formule | Analyse Calc. C% | H% | N% | Analyse Trouvé C% | H% | N% |
|---|---|---|---|---|---|---|---|---|---|---|---|---|---|
| 1 | OH | 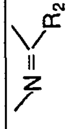 | H | H | H | 225 – 8° | $C_{12}H_7N_3O_3$ | 59.76 | 2.93 | 17.42 | 59.49 | 3.12 | 17.55 |
| 2 | 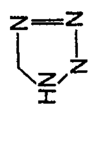 | " | H | H | H | > 300° | *$C_{13}H_8N_8O_2C_3H_4N_2$ | 51.06 | 3.22 | 37.22 | 50.98 | 3.34 | 37.19 |
| 3 | OH | " | Me | Me | Me | 240–3° | $C_{15}H_{13}N_3O_3$ | 63.60 | 4.63 | 14.83 | 63.32 | 4.65 | 14.79 |
| 4 | OH | " | H | Me | Me | 227–30° | $C_{14}H_{11}N_3O_3$ | 62.45 | 4.12 | 15.61 | 62.20 | 4.16 | 15.67 |
| 5 | 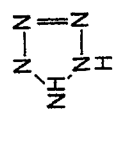 | " | H | Me | Me | > 270° | $C_{15}H_{12}N_8O_2$ | 53.57 | 3.60 | 33.32 | 53.32 | 3.65 | 33.12 |
| 6 | OH | " | H | H | OMe | 235–7° | $C_{13}H_9N_3O_4$ | 57.57 | 3.34 | 15.49 | 57.42 | 3.41 | 15.59 |

* contient 1 mole d'imidazole.

| Exemple | $R_1$ | A | $R_2$ | $R_3$ | $R_4$ | M. pt. | Formule | Analyse Calc. C% | H% | N% | Analyse Trouvé C% | H% | N% |
|---|---|---|---|---|---|---|---|---|---|---|---|---|---|
| 7 | OH | 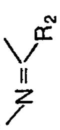 | H | Cl | Cl | 237–40° | $C_{12}H_5N_3O_3Cl_2$ | 45.81 | 1.76 | 13.35 | 45.95 | 1.74 | 13.42 |
| 8 | OH | 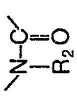 | H | H | H | >300° | $C_{12}H_7N_3O_4$ | 56.04 | 2.74 | 16.34 | 55.76 | 2.87 | 16.40 |
| 9 | 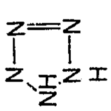 | " | H | H | H | >300° | $C_{13}H_8N_8O_3$ | 48.15 | 2.48 | 34.56 | 48.36 | 3.24 | 34.93 |
| 10 | OH | " | Me | H | H | 216–8° | $C_{13}H_9N_3O_4$ | 57.57 | 3.34 | 15.49 | 57.31 | 3.44 | 15.57 |
| 11 | NHNH2 | " | H | H | H | >300° | $C_{12}H_9N_5O_3$ | 53.14 | 3.34 | 25.82 | 52.27 | 3.34 | 25.89 |

* contient 1 mole d'imidazole.

Exemple 11: 1,5-dioxo 1,6-dihydropyrimido-[1,2-a]quinoxalin-2-carbohydrazide.

On chauffe au bain-marie pendant 15 minutes, un mélange de 500 mg d'acide 1,5-dioxo 1,6-dihydropyrimido[1,2-a]quinoxalin-2-carboxylique et 400 mg de carbonyldiimidazole dans 20 cm³ de diméthylformamide. On refroidit dans un bain de glace puis ajoute 1 cm³ d'une solution renfermant 1,1 g d'hydrate d'hydrazine dans 10 cm³ de diméthylformamide et maintient sous agitation pendant une nuit. On filtre les cristaux formés et les sèche. On obtient 500 mg de produit attendu.

F > 300 °C.

Exemple 12: Composition pharmaceutique.

On a préparé des comprimés répondant à la formule:

| | |
|---|---|
| Produit de l'exemple 8 | 0,015 g |
| Excipient | 0,1 g |

(Détail de l'excipient: lactose, talc, amidon, stéarate de magnésium).

Exemple 13: Composition pharmaceutique.

On a préparé un aérosol délivrant par dose:

| | |
|---|---|
| Produit de l'exemple 8 | 0,002 g |
| Emulsifiant | 0,00015 g |
| Propulseur | 0,050 g |

Etude pharmacologique.

Anaphylaxie cutanée passive (ACP) expérimentale chez le rat.

On induit une anaphylaxie cutanée passive chez des rats mâles Wistar pesant 180–200 g. Les rats ont été sensibilisés par 4 injections intradermiques, au niveau de leur dos préalablement rasé, afin de produire une réaction cutanée passive faisant intervenir des anticorps IgG. (Sensibilisation de 4 heures, par antisérum chauffé pendant une heure à 56 °C).

Le traitement avec l'antigène est effectué ensuite: 1 mg d'ovalbumine est injecté par voie intraveineuse, en même temps que 0,5 cm³ d'une solution à 1% de bleu d'Evans et 30 minutes après, les animaux sont sacrifiés et pour chaque point bleu observé à la surface interne de la peau, une notation est donnée en tenant compte de sa surface et de sa gravité.

Le pourcentage d'inhibition observé après l'administration orale des composés à tester est rapporté dans le tableau ci-après:

| Composé de l'exemple | % inhibition de la réaction cutanée 0,1 mg/kg | 1 mg/kg | 10 mg/kg |
|---|---|---|---|
| 1 | | 9,9 | 50,3 |
| 2 | | 29,9 | 48,6 |
| 8 | 40,5 | 52,4 | 94,1 |
| 9 | 19 | 23 | 35 |
| 10 | | 15,6 | 20,5 |

**Revendications**

1. Nouvelles pyrimidones substituées ainsi que leurs sels, caractérisées en ce qu'elles répondent à la formule générale (I):

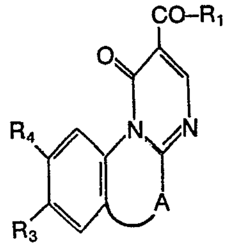

(I)

dans laquelle $R_1$ représente un radical hydroxy, un radical alcoxy renfermant de 1 à 5 atomes de carbone, un radical (1H-tétrazol-5-yl)amino, un radical hydrazino,

A représente un groupement 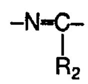

dans lequel $R_2$ représente un atome d'hydrogène ou un radical alcoyle renfermant de 1 à 5 atomes de carbone, étant entendu que $R_2$ ne peut représenter un atome d'hydrogène lorsque $R_1$ représente un radical alcoxy renfermant de 1 à 5 atomes de carbone, ou

A représente un groupement 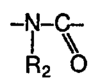

dans lequel $R_2$ a la signification déjà indiquée,

$R_3$, $R_4$, identiques ou différents, représentent un atome d'hydrogène, un atome d'halogène, un radical alcoyle renfermant de 1 à 5 atomes de carbone ou un radical alcoxy renfermant de 1 à 5 atomes de carbone.

2. Dérivés répondant à la formule générale (I) de la revendication 1, ainsi que leurs sels, caractérisés en ce que dans ladite formule (I), $R_1$ et A ont la signification déjà indiquée et $R_3$ et $R_4$ identiques ou différents, représentent un atome d'hydrogène, un atome de chlore, un radical méthoxy ou méthyle.

3. Dérivés selon la revendication 1 ou 2, ainsi que leurs sels, caractérisés en ce que $R_1$ a la signification déjà indiquée,

A représente un groupement 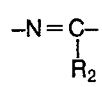

dans lequel $R_2$ représente un atome d'hydrogène ou un radical méthyle étant entendu que $R_2$ ne peut représenter un atome d'hydrogène lorsque $R_1$ représente un radical alcoxy,

$R_3$ et $R_4$, identiques ou différents, représentent un atome d'hydrogène ou de chlore, un radical méthoxy ou méthyle.

4. L'acide 1-oxo 1H-pyrimido[1,2-a]quinoxalin-2-carboxylique et ses sels.

5. Le N-(5-tétrazolyl)-1-oxo 1H-pyrimido[1,2-a]quinoxalin-2-carboxamide et ses sels.

6. Dérivés selon la revendication 1 ou 2, ainsi que leurs sels, caractérisés en ce que $R_1$ a la signification déjà indiquée,

A représente un groupement 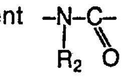

dans lequel $R_2$ représente un atome d'hydrogène ou un radical méthyle,

$R_3$ et $R_4$, identiques ou différents, représentent un atome d'hydrogène ou de chlore, un radical méthoxy ou méthyle.

7. L'acide 1,5-dioxo 1,6-dihydro pyrimido[1,2-a]-quinoxalin-2-carboxylique et ses sels.

8. Procédé de préparation des produits répondant à la formule générale (I) de la revendication 1 ainsi que de leurs sels, caractérisé en ce que l'on fait réagir un produit de formule (II):

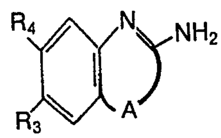

(II)

dans laquelle A, $R_3$ et $R_4$ ont la signification déjà indiquée, avec un produit de formule (III):

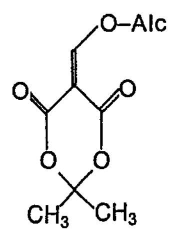

(III)

dans laquelle Alc représente un radical alcoyle renfermant de 1 à 5 atomes de carbone, pour obtenir un produit de formule (IV):

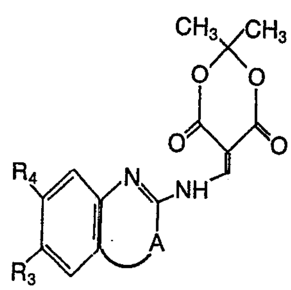

(IV)

dans laquelle A, $R_3$ et $R_4$ ont la signification déjà indiquée, puis cyclise ledit produit de formule (IV), pour obtenir un produit de formule ($I_A$):

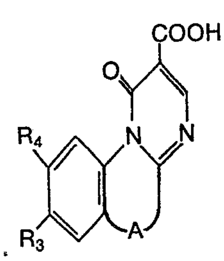

($I_A$)

dans laquelle A, $R_3$ et $R_4$ ont la signification déjà indiquée, et que:

– soit on isole et, si désiré, salifie ledit produit de formule ($I_A$) ainsi obtenu,

– soit on fait réagir ledit produit de formule ($I_A$) ou un dérivé fonctionnel de ce dernier, avec un produit de formule (V):

$$H{-}R'_1 \qquad (V)$$

dans laquelle $R'_1$ a la même signification que R, à l'exclusion du radical hydroxy, pour obtenir un produit de formule ($I_B$):

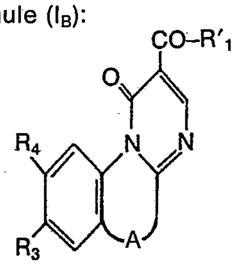

($I_B$)

dans laquelle A, $R'_1$, $R_3$ et $R_4$ ont la signification déjà indiquée, que l'on peut salifier, si désiré.

9. Procédé selon la revendication 8, caractérisé en ce que:

a) la réaction du produit de formule (II) avec le produit de formule (III) est effectuée au sein d'un solvant organique tel que le méthanol;

b) la cyclisation du produit de formule (IV) est effectuée par chauffage du milieu réactionnel au reflux du mélange de 1 à 5 heures;

c) le dérivé fonctionnel du produit de formule ($I_A$) est avantageusement le chlorure d'acide.

10. Variante du procédé de préparation des produits répondant à la formule générale (I) de la revendication 1, dans laquelle $R_3$ et $R_4$ ont la signification déjà indiquée et $R_1$ représente un radical alcoxy renfermant de 1 à 5 atomes de carbone, caractérisée en ce que l'on fait réagir un produit de formule (II):

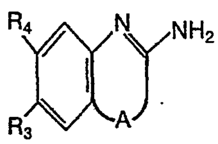

(II)

dans laquelle $R_3$ et $R_4$ ont la signification déjà indiquée, avec un éthoxy méthylène malonate de dialcoyle, pour obtenir un produit de formule (VI):

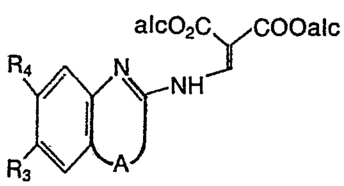

(VI)

dans laquelle A, $R_3$ et $R_4$ ont la signification déjà indiquée et Alc représente un radical alcoyle renfermant de 1 à 5 atomes de carbone, puis cyclise par chauffage le produit de formule (VI) obtenu, pour obtenir le produit de formule I' recherché:

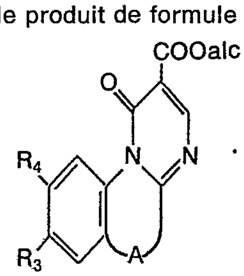

(I')

dans laquelle A, $R_3$, $R_4$ et alc ont la signification déjà indiquée, correspondant à un produit de formule I dans laquelle $R_1$ représente un radical alcoxy, que l'on peut salifier, si désiré.

11. Médicaments, caractérisés en ce qu'ils sont constitués par les nouveaux dérivés tels que définis par la formule (I) de la revendication 1 ainsi que par leurs sels pharmaceutiquement acceptables.

12. Médicaments, caractérisés en ce qu'ils sont constitués par les nouveaux dérivés tels que définis à l'une quelconque des revendications 2, 3, 4, 5, 6 ou 7 ainsi que par leurs sels pharmaceutiquement acceptables.

13. Compositions pharmaceutiques, caractérisées en ce qu'elles renferment à titre de principe actif, l'un au moins des médicaments tels que définis à l'une quelconque des revendications 11 ou 12.

14. A titre de produits industriels nouveaux, les produits de formule (IV):

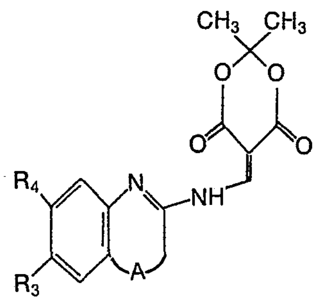

(IV)

dans laquelle A, $R_3$ et $R_4$ ont la signification déjà indiquée à la revendication 1.

## Claims

1. Novel substituted pyrimidones as well as their salts, characterized in that they answer to the general formula (I):

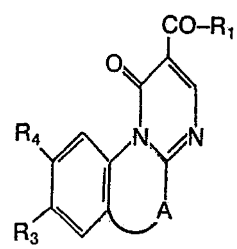

(I)

in which $R_1$ represents a hydroxy radical, an alkoxy radical containing from 1 to 5 carbon atoms, a (1H-tetrazol-5-yl)amino radical or a hydrazino radical,

A represents a group 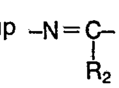

in which $R_2$ represents a hydrogen atom or an alkyl radical containing from 1 to 5 carbon atoms, it being understood that $R_2$ cannot represent a hy-

drogen atom when $R_1$ represents an alkoxy radical containing from 1 to 5 carbon atoms, or

A represents a group 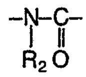

in which $R_2$ has the significance already indicated,

$R_3$ and $R_4$, identical or different, represent a hydrogen atom, a halogen atom, an alkyl radical containing from 1 to 5 carbon atoms or an alkoxy radical containing from 1 to 5 carbon atoms.

2. Derivatives answering to the general formula (I) of Claim 1, as well as their salts, characterized in that in the said formula (I), $R_1$ and A have the significance already given and $R_3$ and $R_4$, identical or different, represent a hydrogen atom, a chlorine atom, a methoxy or a methyl radical.

3. Derivatives according to Claim 1 or 2, as well as their salts, characterized in that $R_1$ has the significance already given,

A represents a group 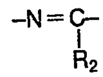

in which $R_2$ represents a hydrogen atom or a methyl radical, it being understood that $R_2$ cannot represent a hydrogen atom when $R_1$ represents an alkoxy radical,

$R_3$ and $R_4$, identical or different, represent a hydrogen or chlorine atom, a methoxy or methyl radical.

4. 1-oxo-1H-pyrimido-[1,2-a]quinoxalin-2-carboxylic acid and its salts.

5. N-(5-tetrazolyl)-1-oxo-1H-pyrimido[1,2-a] quinoxalin-2-carboxamide and its salts.

6. Derivatives according to Claim 1 or 2, as well as their salts, characterized in that $R_1$ has the significance already indicated,

A represents a group 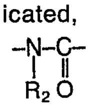

in which $R_2$ represents a hydrogen atom or a methyl radical,

$R_3$ and $R_4$, identical or different, represent a hydrogen or chlorine atom, a methoxy or methyl radical.

7. 1,5-dioxo-1,6-dihydropyrimido[1,2-a]quinoxalin-2-carboxylic acid and its salts.

8. Preparation process for the products answering to the general formula (I) of Claim 1, as well as their salts, characterized in that a product with the formula (II):

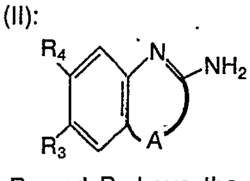

(II)

in which A, $R_3$ and $R_4$ have the significance already indicated, is made to react with a product with the formula (III):

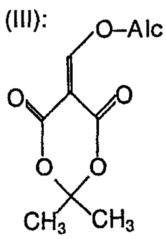

(III)

in which Alk represents an alkyl radical containing from 1 to 5 carbon atoms, so as to obtain a product with the formula (IV):

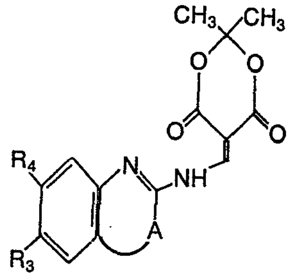

(IV)

in which A, $R_3$ and $R_4$ have the significance already indicated, then the said product with the formula (IV) is cyclized in order to obtain a product with the formula ($I_A$):

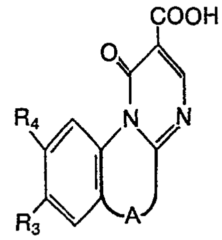

($I_A$)

in which A, $R_3$ and $R_4$ have the significance already indicated, and which:

– either is isolated and, if desired, the said product with the formula ($I_A$) so obtained is salified,

– or the said product with the formula ($I_A$), or a functional derivative of this latter, is made to react with a product with the formula (V):

$$H\text{–}R'_1 \quad (V)$$

in which $R'_1$ has the same significance as R, except for the hydroxy radical, in order to obtain a product with the formula ($I_B$):

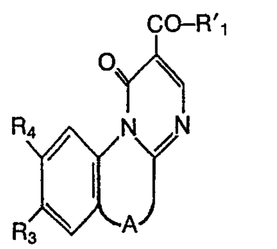

($I_B$)

in which A, $R'_1$, $R_3$ and $R_4$ have the significance already indicated, which can, if desired, be salified.

9. Process according to Claim 8, characterized in that:

a) the reaction of the product with the formula (II) with the product with the formula (III) is carried out in an organic solvent such as methanol;

b) the cyclization of the product with the formula (IV) is carried out by heating the reactional medium to reflux of the mixture for from 1 to 5 hours:

c) the functional derivative of the product with the formula ($I_A$) is advantageously the acid chloride.

10. Variant of the preparation process for the products answering to the general formula (I) of Claim 1, in which $R_3$ and $R_4$ have the significance already indicated and $R_1$ represents an alkoxy radical containing from 1 to 5 carbon atoms, characterized in that a product with the formula (II):

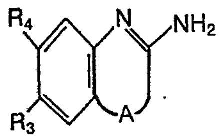

(II)

in which $R_3$ and $R_4$ have the significance already indicated, is made to react with a dialkyl ethoxymethylene malonate, in order to obtain a product with the formula (VI):

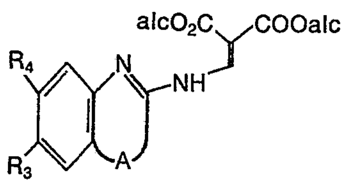

(VI)

in which A, $R_3$ and $R_4$ have the significance already indicated and Alk represents an alkyl radical containing from 1 to 5 carbon atoms, then the product with the formula (VI) obtained is cyclized by heating, so as to obtain the product sought with the formula (I′):

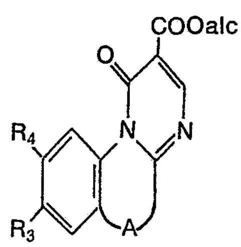

(I′)

in which A, $R_3$, $R_4$ and alk have the significance already indicated, corresponding to a product with the formula I in which $R_1$ represents an alkoxy radical, which can, if desired, be salified.

11. Medicaments, characterized in that they are constituted by the novel derivatives as defined by formula (I) of Claim 1, as well as by the pharmaceutically acceptable salts.

12. Medicaments, characterized in that they are constituted by the novel derivatives as defined in any one of the Claims 1, 3, 4, 5, 6 or 7, as well as by their pharmaceutically acceptable salts.

13. Pharmaceutical compositions, characterized in that they contain as active principle one at least of the medicaments as defined in either of the Claims 11 or 12.

14. As novel industrial products, the products with the formula (IV):

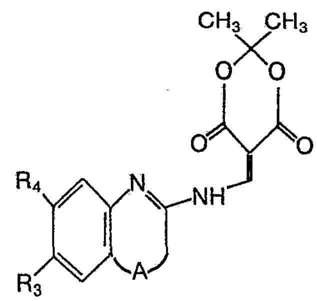

(IV)

in which A, $R_3$ and $R_4$ have the significance already indicated in Claim 1.

**Patentansprüche**

1. Neue substituierte Pyrimidone sowie deren Salze, dadurch gekennzeichnet, dass sie der allgemeinen Formel (I)

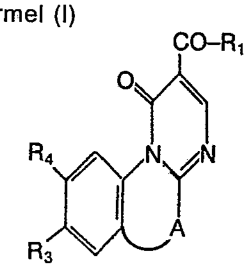

(I)

entsprechen, worin $R_1$ eine Hydroxygruppe, eine Alkoxygruppe mit 1 bis 5 Kohlenstoffatomen, einen (1H-Tetrazol-5-yl)-aminorest oder eine Hydrazinogruppe bedeutet,

A eine Gruppe 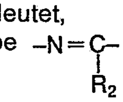

darstellt, worin $R_2$ ein Wasserstoffatom oder einen Alkylrest mit 1 bis 5 Kohlenstoffatomen bedeutet, wobei $R_2$ kein Wasserstoffatom bedeuten kann, wenn $R_1$ einen Alkoxyrest mit 1 bis 5 Kohlenstoffatomen darstellt, oder

A eine Gruppe 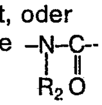

bedeutet, worin $R_2$ die angegebene Bedeutung besitzt,

$R_3$, $R_4$, die gleich oder verschieden sein können, ein Wasserstoffatom, ein Halogenatom, einen Alkylrest mit 1 bis 5 Kohlenstoffatomen oder eine Alkoxygruppe mit 1 bis 5 Kohlenstoffatomen bedeuten.

2. Derivate der allgemeinen Formel (I) gemäss Anspruch 1 sowie deren Salze, dadurch gekennzeichnet, dass in der Formel (I) $R_1$ und A die abgegebene Bedeutung besitzen und $R_3$ und $R_4$, die gleich oder verschieden sind, ein Wasserstoffatom, ein Chloratom, einen Methoxy- oder Methylrest bedeuten.

3. Derivate gemäss Anspruch 1 oder 2 sowie deren Salze, dadurch gekennzeichnet, dass $R_1$ die angegebene Bedeutung besitzt

A eine Gruppe 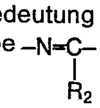

darstellt, worin $R_2$ ein Wasserstoffatom oder einen Methylrest bedeutet, wobei $R_2$ kein Wasserstoffatom bedeuten kann, wenn $R_1$ einen Alkoxyrest bedeutet,

$R_3$, $R_4$, die gleich oder verschieden sind, ein Wasserstoff- oder Chloratom, eine Methoxy- oder Methylgruppe bedeuten.

4. 1-Oxo-1H-pyrimido-[1,2-a]-chinoxalin-2-carbonsäure und deren Salze.

5. N-(5-Tetrazolyl)-1-oxo-1H-pyrimido-[1,2-a]-chinoxalin-2-carboxamid und dessen Salze.

6. Derivate gemäss Anspruch 1 oder 2 sowie deren Salze, dadurch gekennzeichnet, dass $R_1$ die angegebene Bedeutung besitzt,

A eine Gruppe 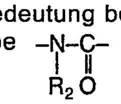

worin $R_2$ ein Wasserstoffatom oder einen Methylrest bedeutet, ist,

$R_3$, $R_4$, die gleich oder verschieden sind, ein Wasserstoff- oder Chloratom, einen Methoxy- oder Methylrest bedeuten.

7. 1,5-Dioxo-1,6-dihydro-pyrimido-[1,2-a]-chinoxalin-2-carbonsäure und deren Salze.

8. Verfahren zur Herstellung von Produkten der allgemeinen Formel (I) gemäss Anspruch 1 sowie von deren Salzen, dadurch gekennzeichnet, dass man ein Produkt der Formel (II)

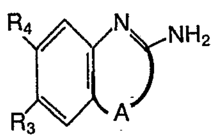

(II)

worin A, $R_3$ und $R_4$ die angegebene Bedeutung besitzen, mit einem Produkt der Formel (III)

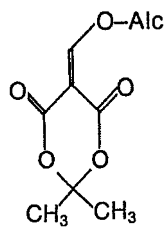

(III)

worin Alc einen Alkylrest mit 1 bis 5 Kohlenstoffatomen bedeutet, umsetzt, um ein Produkt der Formel (IV)

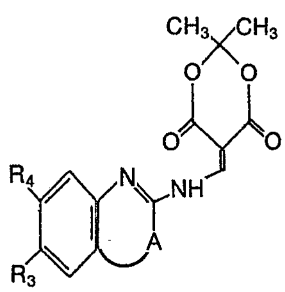

(IV)

zu erhalten, worin A, $R_3$ und $R_4$ die angegebene Bedeutung besitzen, danach das Produkt der Formel (IV) cyclisiert, um ein Produkt der Formel ($I_A$)

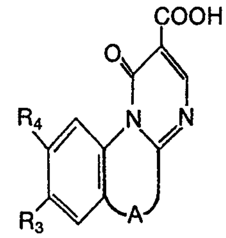

($I_A$)

zu erhalten, worin A, $R_3$ und $R_4$ die angegebene Bedeutung besitzen, und

– entweder das so erhaltene Produkt der Formel ($I_A$) isoliert und gewünschtenfalls in ein Salz überführt,

– oder das Produkt der Formel ($I_A$) oder ein funktionelles Derivat dieses letzteren mit einem Produkt der Formel (V)

$$H\text{–}R'_1 \qquad (V)$$

umsetzt, worin $R'_1$ die für R angegebene Bedeutung mit Ausnahme eines Hydroxyrestes besitzt, um ein Produkt der Formel ($I_B$)

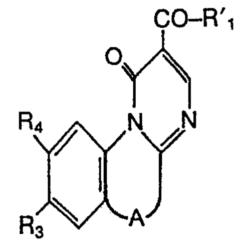

($I_B$)

zu erhalten, worin A, $R'_1$, $R_3$ und $R_4$ die angegebene Bedeutung besitzen, das man gewünschtenfalls in ein Salz überführen kann.

9. Verfahren gemäss Anspruch 8, dadurch gekennzeichnet, dass

a) die Umsetzung des Produkts der Formel (II) mit dem Produkt der Formel (III) in dem Medium eines organischen Lösungsmittels, wie Methanol, durchgeführt wird,

b) die Cyclisierung des Produkts der Formel (IV) durch Erhitzen des Reaktionsmilieus unter Rückfluss des Gemisches während 1 bis 5 h durchgeführt wird,

c) das funktionelle Derivat des Produkts der Formel ($I_A$) vorteilhafterweise das Säurechlorid ist.

10. Abänderung des Verfahrens zur Herstellung der Produkte der allgemeinen Formel (I) gemäss Anspruch 1, worin $R_3$ und $R_4$ die angegebene Bedeutung besitzen und $R_1$ eine Alkoxygruppe mit 1 bis 5 Kohlenstoffatomen bedeutet, dadurch gekennzeichnet, dass man ein Produkt der Formel (II)

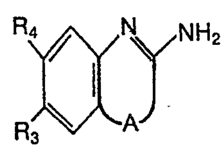

(II)

worin $R_3$ und $R_4$ die angegebene Bedeutung besitzen, mit einem Dialkyläthoxymethylenmalonat umsetzt, um ein Produkt der Formel (VI)

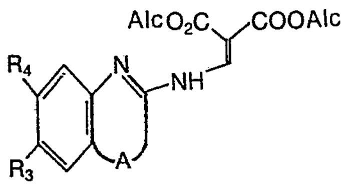

(VI)

zu erhalten, worin A, $R_3$ und $R_4$ die angegebene Bedeutung besitzen, und Alc einen Alkylrest mit 1 bis 5 Kohlenstoffatomen darstellt, danach durch Erhitzen des erhaltenen Produkts der Formel (VI) cyclisiert, um das gewünschte Produkt der Formel (I')

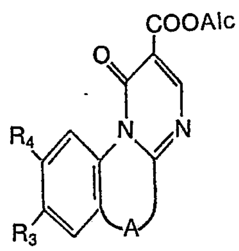

(I')

zu erhalten, worin A, $R_3$, $R_4$ und Alc die angegebene Bedeutung besitzen, entsprechend einem Produkt der Formel (I), worin $R_1$ einen Alkoxyrest bedeutet, den man gewünschtenfalls in ein Salz überführen kann.

11. Arzneimittel, dadurch gekennzeichnet, dass sie aus den neuen Derivaten der Formel (I) gemäss Anspruch 1 sowie aus deren pharmazeutisch verträglichen Salzen bestehen.

12. Arzneimittel, dadurch gekennzeichnet, dass sie aus den neuen Derivaten gemäss einem der Ansprüche 2, 3, 4, 5, 6 oder 7 sowie aus deren pharmazeutisch verträglichen Salzen bestehen.

13. Pharmazeutische Zusammensetzungen, dadurch gekennzeichnet, dass sie als Wirkstoff zumindest eines der Arzneimittel gemäss einem der Ansprüche 11 oder 12 enthalten.

14. Als neue industrielle Produkte die Produkte der Formel (IV)

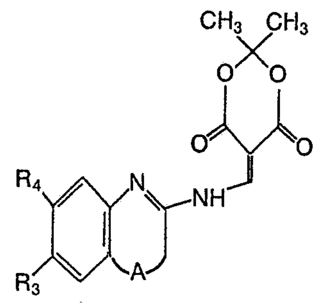

(IV)

worin A, $R_3$ und $R_4$ die in Anspruch 1 angegebene Bedeutung besitzen.